# EUROPÄISCHE PATENTSCHRIFT

(11) **EP 1 609 464 B1**
(45) Veröffentlichungstag und Bekanntmachung des Hinweises auf die Patenterteilung: **12.08.2009**
(21) Anmeldenummer: 05104906.2
(22) Anmeldetag: 06.06.2005
(51) Int. Cl.: A61Q 19/10, A61K 8/04, A61K 8/365, A61K 8/44, A61K 8/60

(54) **Produkte zur Reinigung der Intimregion**
Intimate hygiene products
Produits d'hygiène intime

(30) Priorität: 25.06.2004 DE 102004031668
(43) Veröffentlichungstag der Anmeldung: 28.12.2005
(73) Patentinhaber: Beiersdorf AG, 20245 Hamburg (DE)
(72) Erfinder: Kuether, Joerg, 25469, Halstenbek (DE); Lenuck, Andreas, 22527, Hamburg (DE)

(56) Entgegenhaltungen:
- EP-A- 1 302 191
- DE-A1- 10 007 321
- DE-A1- 10 148 392
- US-A- 3 962 150
- US-A- 6 053 364
- US-A1- 2002 045 554

## Beschreibung

Die vorliegende Erfindung betrifft Produkte zur Reinigung der Intimregion, insbesondere solche, die tensidische und schäumende Zubereitungen umfassen und die das Wachstum von Lactobacilli oder Doederlein-Bakterien nur in geringem Umfang beeinträchtigt.
Die Vaginalschleimhaut wird im gesunden Zustand von Lactobacilli oder Doederlein-Bakterien besiedelt. Diese bewahren die Schleimhaut vor Infektionen von schädlichen Mikroorganismen, indem sie mit diesen um Nahrung (Schleimhautpolysaccharide) konkurrieren und Milchsäure absondern. Das milchsaure Milieu auf der Schleimhaut führt zu einem pH-Wert von 3,8 bis 4,6 und hemmt das Wachstum schädlicher Mikroorganismen. Der pH-Wert der Vaginalschleimhaut sollte durch die Reinigung der Intimregion möglichst nicht beeinflusst werden, um Infektionen durch schädliche Mikroorganismen zu verhindern.

Übliche Zuberreitungen zur Körperreinigung weisen aber meist höhere pH-Werte auf: Seife ist alkalisch (pH 9-11), auch auf den pH-Wert der Säureschutzmantel der gewöhnlichen Haut angepasste Zubereitungen weisen pH-Werte größer 5 auf. Alle diese Zubereitungen würden also in den Säure-Base-Haushalt der Vaginalschleimhaut stark eingreifen.
Um dieses Problem zu lösen gab es in der Vergangenheit zahlreiche Lösungsversuche. Bekannt ist ein kosmetisches Reinigungsmittel, welches eine Kombination aus Pack- und Applikationsmittel und einer flüssigen Reinigungszubereitung ist und mit Hilfe des Applikators in einen stabilen Schaum verwandelt wird.

Schäume sind Gebilde aus gasgefüllten, kugel- oder polyederförmigen Zellen, welche durch flüssige, halbflüssige, hochviskose oder feste Zellstege begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum. Auch Schäume sind thermodynamisch instabil, da durch Verkleinerung der Oberfläche Oberflächenenergie gewonnen werden kann. Die Stabilität und damit die Existenz eines Schaums ist somit davon abhängig, wieweit es gelingt, seine Selbstzerstörung zu verhindern.

Kosmetische Schäume sind in der Regel dispergierte Systeme aus Flüssigkeiten und Gasen, wobei die Flüssigkeit das Dispergiermittel und das Gas die dispergierte Substanz darstellen. Schäume aus niedrigviskosen Flüssigkeiten werden temporär durch oberflächenaktive Substanzen (Tenside, Schaumstabilisatoren) stabilisiert. Solche Tensidschäume haben aufgrund ihrer großen inneren Oberfläche ein starkes Adsorptionsvermögen, welches beispielsweise bei Reinigungs- und Waschvorgängen ausgenutzt wird. Dementsprechend finden kosmetische Schäume insbesondere in den Bereichen der Reinigung, beispielsweise als Rasierschaum, und der Haarpflege Verwendung.

Zur Erzeugung von Schaum wird Gas in geeignete Flüssigkeiten eingeblasen, oder man erreicht die Schaumbildung durch heftiges Schlagen, Schütteln, Verspritzen oder Rühren der Flüssigkeit in der betreffenden Gasatmosphäre, vorausgesetzt, dass die Flüssigkeiten geeignete Tenside oder andere grenzflächenaktive Stoffe (sogenannte Schaumbildner) enthalten, die außer Grenzflächenaktivität auch ein gewisses Filmbildungsvermögen besitzen.

Kosmetische Schäume haben gegenüber anderen kosmetischen Zubereitungen den Vorteil, dass sie eine feine Verteilung von Wirkstoffen auf der Haut erlauben. Allerdings sind kosmetische Schäume in der Regel nur durch Verwendung besonderer Tenside, welche darüber hinaus oft wenig hautverträglich sind, zu erreichen.

Eine Anforderung an kosmetische Zubereitungen ist aber, dass diese eine möglichst jahrelange Stabilität besitzen. Diesem Problem wird im allgemeinen dadurch Rechnung getragen, dass der Verbraucher den eigentlichen Schaum erst bei der Anwendung mit Hilfe eines geeigneten Sprühsystems selbst erzeugt, wozu beispielsweise Sprühdosen verwendet werden können, in denen ein verflüssigtes Druckgas als Treibgas dient. Beim Öffnen des Druckventils entweicht das Treibmittel-Flüssigkeitsgemisch durch eine feine Düse, das Treibmittel verdampft und hinterlässt einen Schaum.

Die Herstellung von Reinigungschäumen für die Körperpflege mit Hilfe eines Pumpschäumers (Pumpfoamer, US 6053364) der Fa. Airspray ist bekannt. Als Tensidlösungen kamen aber bisher nur Lösungen mit einer Kombination aus amphoteren und anionischen Tensiden (WO 99/39689) oder Kombinationen aus nichtionischen und amphoteren Tensiden (US 5635469) zum Einsatz. Erstere Ausführung schließt den Einsatz von wasserunlöslichen Bestandteilen explizit aus, während Zweitere kationische oder nichtionische Polymere als "Hautkonditionierer" enthält.

Der Vorteil der Schaumbereitung mit einem Pumpschäumer (Pumpfoamer, US 6053364) - gegenüber der herkömmlichen Schaumbereitung durch Verreiben mit den Händen - liegt in der geringen Konzentration an Tensiden, die benötigt wird um einen cremigen, feinporigen Schaum zu erhalten. Dadurch sind die so gewonnenen Reinigungsschäume für die Reinigung empfindlicher Haut besonders geeignet.

Bisherige Pumpschäumer weisen jedoch den Nachteil der Verkeimung des Füllgutes auf, da bei der Betätigung - dem Pumpvorgang - geringe Mengen von sich im Bereich des Pumpkopfes befindlichen Spritzwassers in das Pumpsystem eingesaugt werden können.

Die Schaumbildung wird hervorgerufen durch mechanische Pumpsysteme - wie in WO 00/78629 A1 beschrieben - in denen die flüssige Tensidzubereitung zur feinporigen Luftanreicherung mit hoher Geschwindigkeit durch ein siebartiges Gewebe oder eine ähnliche Struktur geführt wird, um die gewünschte Schaumbildung zu erzielen. Eine konstruktive Veränderung von bekannten Pumpfoamem kann das eindringen (ansaugen) von Spritzwasser und damit die Verkeimungsproblematik so stark reduzieren, das es nahezu keines Zusatzes von Desinfektionsmittel/Konservierungsmitteln/Bakteriziden zur Tensidzubereitung bedarf, was wiederum ein entscheidender Vorteil bei der Hautverträglichkeit darstellt. Der Spitzwasserschutz an dem Schaumpumpsystem wird herbeigeführt indem die Belüftungsöffnungen oder -Kanäle durch einen schild- oder dachartigen Kragen geschützt sind, so dass aufspritzendes Wasser an den Belüftungsöffnungen vorbeigeführt wird. Ferner ist er Kragen so ausgeformt, dass er ein Eindringen des Spritzwassers in die Innenkonstruktion sicher verhindert bzw. von der sensiblen Innenkonstruktion ableitet, so dass ein Kontakt des mikrobiologisch belasteten Spritwassers mit der Tensidzubereitung in jeglicher Form ausgeschlossen ist.

Die Schrift DE 10148392 offenbart ein kosmetisches Reinigungsprodukt umfassend eine schäumbare, wässrige Reinigungszubereitung, enthaltend ein oder mehrere nichtionische Tenside und ein oder mehrere anionische Tenside, und einen Pumpschäumer, enthaltend einen Vorratsbehälter und einen als Verschluss ausgebildeten Pumpmechanismus mit Steigrohr zur Aufschäumung der sich im Vorratsbehälter befindenden Tensidlösung.

Die Schrift US 3962150 offenbart wässrige, nicht unter Druck stehende, schaumbildende Hautreinigungszubereitungen bestehend aus 0,5 - 14,5% nichtionischen oberflächenaktiven Stoffen, 0,5 - 14,5% anionischen oberflächenaktiven Stoffen und 1-15% bestimmter alkoholischer Lösungsmittel und 70-98% Wasser.

Die Schrift EP 728475 offenbart ein Hautreinigungsprodukt umfassend einen zum Aufschäumen geeigneten Behälter mit einer porösen Membran sowie eine Zubereitung mit bestimmten anionischen oberflächenaktiven Stoffen und mit nichtionischen oberflächenaktiven Stoffen.

DE 100 07 321 offenbart ein Schaumreinigungsprodukt zur Körperpflege, bestehend aus einem wässrigen Reinigungsmittel und einem treibgasfreien Spendebehälter mit Schaumventil, **dadurch gekennzeichnet, dass** das Reinigungsmittel wenigstens 5-20 Gew.-% eines oberflächlichenaktiven Sulfobernsteinsäuremonoestersalzes 1-10 Gew.-% eines oberflächenaktiven Ampho- oder Betaintensids und 60-90 Gew.-% Wasser enthält.

Bisherige Intimreinigungsprodukte basieren auf Standardreinigungsformulierungen mit hohen Tensidkonzentrationen und allenfalls leicht adaptierten pH Werten. Diese können insbesondere bei häufiger Anwendung das natürliche Schleimhautgleichgewicht stören und Infektionen der Vaginalregion begünstigen.

Davon ausgehend lag der vorliegenden Erfindung die Aufgabe zugrunde, ein sehr mildes und dennoch ausreichend schäumendes an die Bedingungen der Intimregion angepasstes tensidisches Reinigungsprodukt zu finden, das eine gute Reinigung unter geringster Störung des Säure-Basegleichgewichtes der Vaginalschleimhaut erlaubt.

Es hat sich für den Fachmann nicht vorhersehbar herausgestellt, dass ein kosmetisches Reinigungsprodukt umfassend
a) eine flüssigen Reinigungsformulierung mit einem pH Wert kleiner 5,0 enthaltend:
   - mindestens ein anionisches Tensid,
   - ein oder mehrere nichtionische Tenside,
   - 0,001- 5% Milchsäure,
b) eine Verpackungssystem in Form eines Pumpschäumers enthaltend einen Vorratsbehälter und einen als Verschluss ausgebildeten Pumpmechanismus mit Steigrohr zur Aufschäumung der sich im Vorratsbehälter befindenden Tensidlösung, Tensidlösung wobei, dass das anionische Tensid aus der Gruppe der Acylglutamate gewählt wird den Mängeln des Standes der Technik abhelfen.

Durch die Kombination eines speziellen Packmittels mit reduzierter Ausbringungsmenge (Pumpfoamer) und einer speziellen Reinigungsformulierung (niedriger pH-Wert und Milchsäure) gelingt es, eine besonders milde Reinigung bei gleichzeitig für den Verbraucher ansprechender Schaummenge der Intimregion zu ermöglichen.
Die für diesen Anwendungsbereich neue Kombination einem Pumpspender, der es erlaubt kleine Menge sehr milder Tenside sowie Milchsäure mit gleichzeitig viel Schaum zu erzeugen (Der Anwender bringt bei gleicher Schaummenge wesentlich weniger Tensid auf die Haut auf) ermöglicht nun eine schonende Reinigung so dass die vorhandene Vaginalflora (insbesondere Lactobacillus) weitestgehend unbeeinträchtigt bleibt. Es handelt sich also um eine milde, auf die biologische Flora der Intimregion abgestimmte Reinigungsformulierung.

Als nichtionische/s Tensid/e werden vorzugsweise verwendet
1. Alkohole,
2. Alkanolamide, wie Cocamide MEA/DEA/MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/propoxylierte Ester, ethoxylierte/propoxylierte Glycerinester, ethoxylierte/propoxylierte Cholesterine, ethoxylierte/propoxylierte Triglyceridester, ethoxyliertes propoxyliertes Lanolin, ethoxylierte/propoxylierte Polysiloxane, propoxylierte POE-Ether und Alkylpolyglycoside wie Laurylglucosid, Decylglycosid und Cocoglycosid.
6. Sucroseester, -Ether
7. 7 Polyglycerinester, Diglycerinester, Monoglycerinester
8. Methylglucosester, Ester von Hydroxysäuren

Bevorzugt ist es, wenn der Pumpschäumer spritzwassergeschützt ausgeführt ist. Unter Spritzwasserschutz im Sinne dieser Anmeldung sind Ausführungen des Packmittels zu verstehen in denen durch Dichtungssysteme oder mechanische Vorkehrungen das Eindringen von externem Wasser in das sich im Inneren der Packung befindliche Füllgut minimiert oder vollständig untenbunden wird.
Besonders bevorzugt sind die Pumpschäumer der Fa. Airspray International B. V., besonders bevorzugt der spritzwassergeschützte Pumpschäumer (Airspray International B. V., Typ WR3).

Weiter bevorzugt ist es, wenn der Pumpschäumer an einem eingebauten Netz bei der Entnahme Schaum erzeugt.

Es haben sich die Alkylpolyglukoside, im besonderen Laurylglucosid, Decylglycosid und Cocoglycosid als vorteilhaft erwiesen.

Bevorzugt ist es daher, wenn das nichtionische Tensid aus der Gruppe der Alkylglukoside gewählt wird, besonders bevorzugt ist es, wenn Lauryl- und/oder Decylglucosid gewählt wird. Das anionische Tensid wird aus der Gruppe der Acylglutamate gewählt, bevorzugt wird Natrium Cocoylglutamat gewählt.

Besonders vorteilhaft sind Tensidkonzentrationen die sich im Bereich von 2 bis 15 Gew-%, insbesondere im Bereich von 4 bis 9 Gew-% (immer bezogen auf das Gesamtgewicht der Formulierung) bewegen.

Das erfindungsgemäße Verhältnis vom anionischen zum nichtionischen Tensid soll wie a zu b - mit a und b einer rationalen Zahl zwischen 2 und 5 sein. Als bevorzugt hat sich ein Verhältnis vom anionischen zum nichtionischen Tensid von 5 zu 3 bis 3,5 zu 4, besonders bevorzugt von 2 zu 3 herausgestellt.

Weiter bevorzugt ist es, wenn die Reinigungslösung Natrium Cocoylglutamat und Decylglucosid in einem Verhältnis von 4:1 bis 0,5:1, bevorzugt von 3:1 bis 0,5:1, besonders bevorzugt von 2:1 bis 0,7:1 enthält.

Weiter bevorzugt ist es, wenn die Reinigungslösung hautberuhigende Hilfstoffe, bevorzugt Polydocanol, Panthenol, Nachtkerzenöl, Bisabolol, Kamillenextrakt enthält, besonders bevorzugt 0,001- 5 gew.% Kamilenextrakt enthält.

Weiter ist es bevorzugt, wenn eine erfindungsgemäße Reinigungszubereitung weitere kosmetische Hilfsstoffe enthält, insbesondere Emolients, Ölkomponenten, Farbstoffe, Pigmente, Konservierungsmittel und Parfüm, besonders bevorzugt hydrierte Fettsäuremonoglyceride, Fettsäurediglyceride und/oder Fettsäuretriglyceride enthält, die ethoxyliert wurden und einen Ethoxylierungsgrad von 20 bis 500 aufweisen.

Hinsichtlich der Viskosität erfindungsgemäßer Reinigungsprodukte ist es bevorzugt, die Reinigungsformulierung eine Viskosität von 1 bis 1500 mPas, besonders bevorzugt von 1 bis 1000 mPas ganz besonders bevorzugt von 1 bis 500 mPas, gemessen mit Viskotester der Firma Thermo Haake GmbH, Modell Viskotester VT 02, Drehkörper Typ 1 (Messung erfolgt analog zu DIN 53019 und DIN 168), aufweist.

Ganz besonders bevorzugt ist es, wenn die Reinigungsformulierung einphasig ist.
Die Erfindung umfasst auch die Verwendung einer erfindungsgemäßen Reinigungszubereitung zur Reinigung der Vaginalschleimhaut.

Als hautpflegende Emolients können Öle zum Einsatz kommen. Eine gegebenenfalls gewünschte Ölkomponente der kosmetischen oder dermatologischen Reinigungszubereitungen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölkomponente vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölkomponente gewählt aus der Gruppe 2-Ethylhexylisostearat, Octyldodecanol, Isotridecylisbnonanoat, Isoeicosan, 2-Ethylhexylcocoat, C12-15-Alkylbenzoat, Capryl-Caprinsäure-triglycerid, Dicaprylylether.

Besonders vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölkomponente ferner einen Gehalt an cyclischen oder linearen Silikonölen aufweisen oder vollständig aus solchen Ölen bestehen, wobei allerdings bevorzugt wird, ausser dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäss zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölkomponente wird ferner vorteilhaft aus der Gruppe der Phospholipide gewählt. Die Phospholipide sind Phosphorsäureester acylierter Glycerine. Von grösster Bedeutung unter den Phosphatidylcholinen sind beispielsweise die Lecithine.

Bevorzugt im Sinne der Erfindung für die Verwendung dieser Ölkomponenten ist die Kombination mit Solubilisatoren, im Besonderen in der Form der hydrierten Fettsäuremonoglyceride, Fettsäurediglyceride und/oder Fettsäuretriglyceride, die ethoxyliert wurden und einen Ethoxylierungsgrad von 20 bis 500 aufweisen. Im Besonderen haben sich die Rohstoffe PEG-200 hydriertes Glycerylpalmitat, PEG-100 hydriertes Glycerylpalmitat und PEG-40 hydriertes Rizinusöl als vorteilhaft erwiesen.

Durch die Kombination von Polymeren und den zuvor genannten Wirk- und Hilfsstoffen, lässt sich die Wirkung verstärken und ein noch besseres, zuvor nicht erreichtes Hautgefühl erreichen.

Von solchen Polymeren mit wenigstens teilweise quaternisierten Stickstoffgruppen (im folgenden "Filmbildner" genannt), eigenen sich bevorzugt solche, welche gewählt werden aus der Gruppe der Substanzen, welche nach der INCI-Nomenklatur (International Nomenclature Cosmetic Ingredient) den Namen "Polyquaternium" tragen, beispielsweise:
Polyquaternium-2 (Chemical Abstracts-Nr. 63451-27-4, z. B. Mirapol TM A-15)
Polyquaternium-5 (Copolymeres aus dem Acrylamid und dem beta - Methacryloxyethyltrimethylammoniummethosulfat, CAS-Nr. 26006-22-4)
Polyquaternium-6 (Homopolymer des N,N-Dimethyl-N-2-propenyl-2-propen-1- aminiumchlorids, CAS-Nr. 26062-79-3, z. B. Merquat TM 100
Polyquaternium-7 N,N-Dimethyl-N-2-propenyl-2-propen-1-aminiumchlorid, Polymeres mit 2-Propenamid, CAS-Nr. 26590-05-6, z. B. Merquat TM S
Polyquaternium-10 Quaternäres Ammoniumsalz der Hydroxyethylcellulose, CAS-Nr. 53568-66-4, 55353-19-0, 54351-50-7, 68610-92-4, 81859-24-7, z. B. Celquat TM SC-230M,

Polyquaternium-11 Vinylpyrrolidon/dimethylaminoethyl-Methacrylat-Copolymer/Diethylsulfat-Reaktionsprodukt, CAS-Nr. 53633-54-8, z. B. Gafquat TM 755N Polyquaternium-16 Vinylpyrrolidon/vinylimidazoliniummethochlorid-Copolymer, CAS-Nr. 29297-55-0, z. B. Luviquat TM HM 552
Polyquaternium-17 CAS-Nr. 90624-75-2, z. B. Mirapol TM AD-1
Polyquaternium-19 Quaternisierter wasserlöslicher Polyvinylalkohol
Polyquaternium-20 in Wasser dispergierbarer quaternisierter Polyvinyloctadecylether Polyquaternium-21 Polysiloxan-polydimethyl-dimethylammoniumacetat-Copolymeres, z. B. Abil TM B 9905
Polyquaternium-22 Dimethyldiallylammoniumchlorid/Acrylsäure-Copolymer, CAS-Nr. 53694-7-0, z. B. Merquat TM 280
Polyquaternium-24 Polymeres quaternäres Ammoniumsalz der Hydroxyethylcellulose, Reaktionsprodukt mit einem mit Lauryldimethylammonium substituierten Epoxid, CAS-Nr. 107987-23-5, z. B. Quatrisoft TM LM-200
Polyquaternium-28 Vinylpyrrolidon/Methacrylamidopropyltrimethylammoniumchlorid- Copolymer, z. B. Gafquat TM HS-100
Polyquaternium-29 z. B. Lexquat TM CH
Polyquaternium-31 CAS-Nr. 136505-02-7, z. B. Hypan TM QT 100
Polyquaternium-32 N,N,N-trimethyl-2-[(2-methyl-1-oxo-2-propenyl)oxy]-Ethanaminiumchlorid, polymer mit 2-Propenamid, CAS-Nr. 35429-19-7
Polyquaternium-37 CAS-Nr. 26161-33-1

Bevorzugt haben sich die Polymere Polyquaternium-10 und Polyquaternium-22 als vorteilhaft erwiesen.

Die erfindungsgemäßen kosmetischen und dermatologischen Zubereitungen können kosmetische Hilfsstoffe enthalten, wie sie üblicherweise in solchen Zubereitungen verwendet werden, z. B. Konservierungsmittel, Bakterizide, Viruzide, Parfüme, Farbstoffe, Pigmente, die färbende Wirkung haben, weitere, nicht unter die Definition der erfindungsgemäßen Verdicker fallende Verdickungsmittel, oberflächenaktive Substanzen, Emulgatoren, weichmachende, anfeuchtende und/oder feuchthaltende Substanzen, entzündungshemmende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel.

Das Weglassen eines einzelnen Bestandteiles beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

Die Beispiele 1,5,10,11,13 and 15 sind

### nicht anspruchsgemäß.

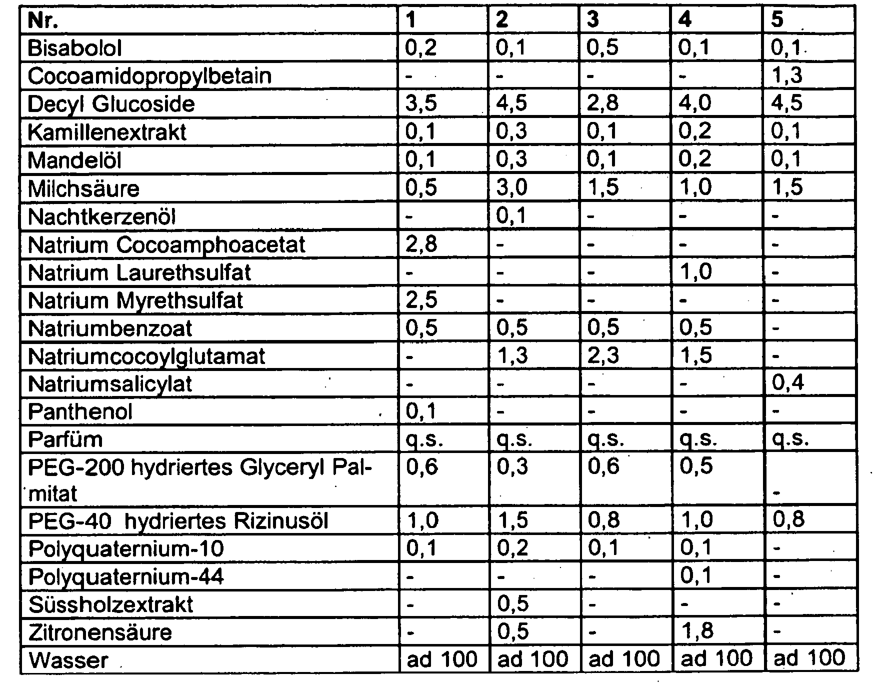

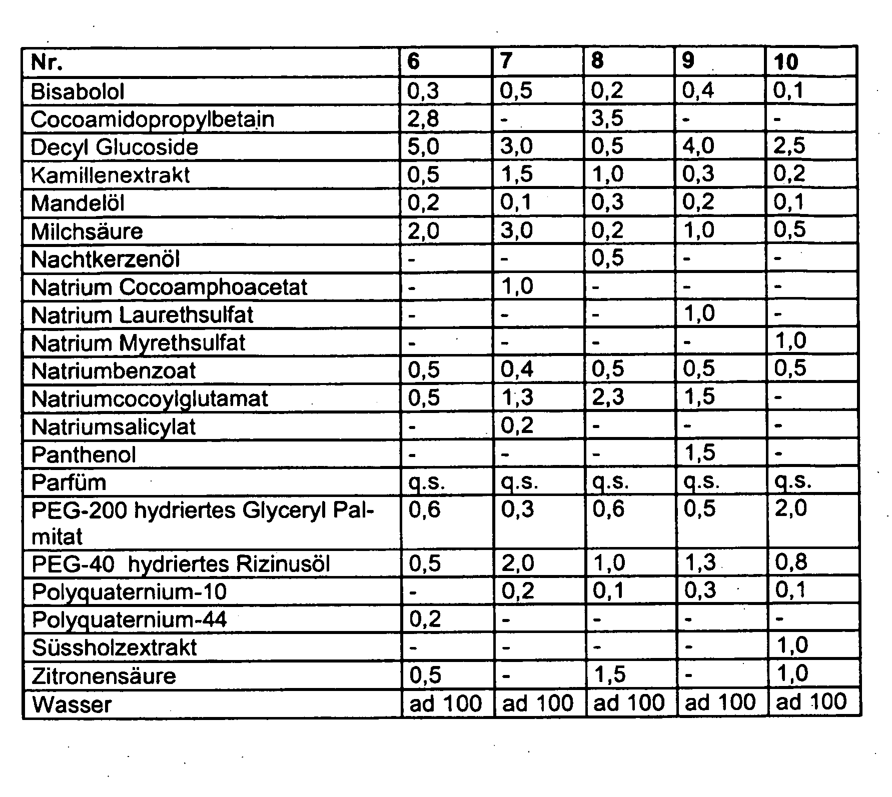

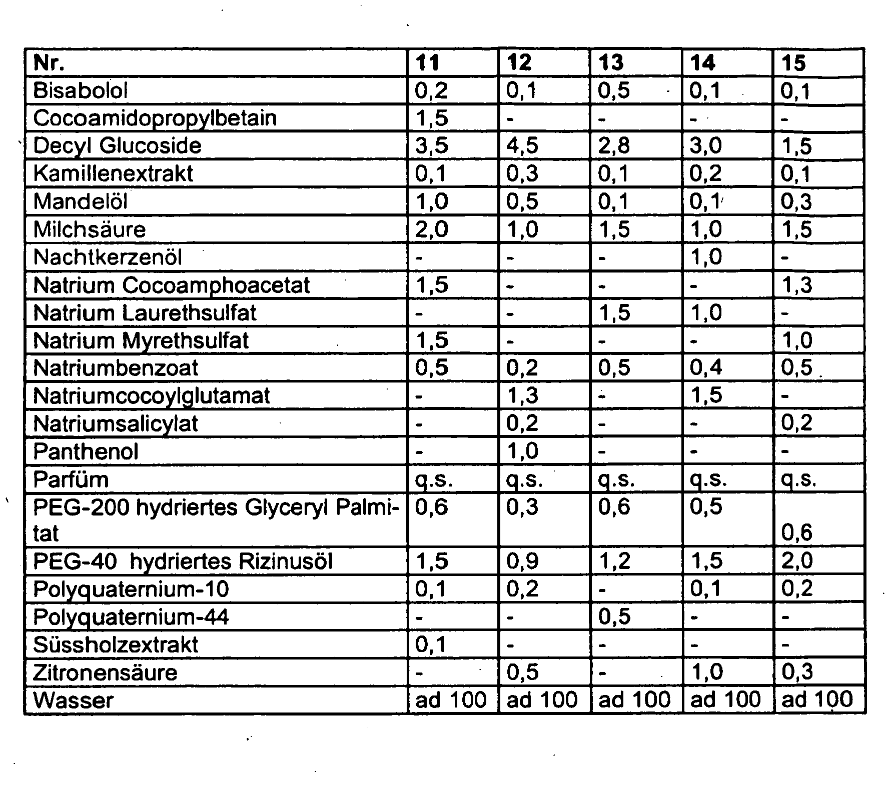

## Patentansprüche

1. Kosmetisches Reinigungsprodukt umfassend
a) eine flüssigen Reinigungsformulierung mit einem pH Wert kleiner 5,0 enthaltend:
- mindestens ein anionisches Tensid,
- ein oder mehrere nichtionische Tenside,
- 0,001- 5% Milchsäure,
b) eine Verpackungssystem in Form eines Pumpschäumers enthaltend einen Vorratsbehälter und einen als Verschluss ausgebildeten Pumpmechanismus mit Steigrohr zur Aufschäumung der sich im Vorratsbehälter befindenden Tensidlösung **dadurch gekennzeichnet, dass** das anionische Tensid aus der Gruppe der Acylglutamate gewählt wird.

2. Reinigungsprodukt nach Anspruch 1 **dadurch gekennzeichnet, dass** der Pumpschäumer spritzwassergeschützt ausgeführt ist.

3. Reinigungsprodukt nach einem der vorangehenden Ansprüche **dadurch gekennzeichnet, dass** der Pumpschäumer an einem eingebauten Netz bei der Entnahme Schaum erzeugt.

4. Reinigungsprodukt nach einem der vorangehenden Ansprüche **dadurch gekennzeichnet, dass** das nichtionische Tensid aus der Gruppe der Alkylglukoside gewählt wird.

5. Reinigungsprodukt nach einem der vorangehenden Ansprüche **dadurch gekennzeichnet, dass** als nichtionisches Tensid Lauryl- und/oder Decylglucosid gewählt wird.

6. Reinigungsprodukt nach einem der vorangehenden Ansprüche **dadurch gekennzeichnet, dass** als anionisches Tensid Natrium Cocoylglutamat gewählt wird.

7. Reinigungsprodukt nach einem der vorangehenden Ansprüche **dadurch gekennzeichnet, dass** die Tensidkonzentration im Bereich von 2 bis 15 Gew.-% bezogen auf das Gesamtgewicht der Formulierung, insbesondere im Bereich von 4 bis 9 Gew.-% liegt.

8. Reinigungsprodukt nach einem der vorangehenden Ansprüche **dadurch gekennzeichnet, dass** das Verhältnis vom anionischen zum nichtionischen Tensid wie a zu b - mit a und b einer rationalen Zahl zwischen 2 und 5 - ist, bevorzugt das Verhältnisse 5 : 3 bis 3,5 : 4 beträgt und ganz besonders 2,0 : 3 beträgt.

9. Reinigungsprodukt nach einem der vorangehenden Ansprüche **dadurch gekennzeichnet, dass** die Reinigungslösung Natrium Cocoylglutamat und Decylglucosid in einem Verhältnis von 4:1 bis 0,5:1, bevorzugt von 3:1 bis 0,5:1, besonders bevorzugt von 2:1 bis 0,7:1 enthält.

10. Reinigungsprodukt nach einem der vorangehenden Ansprüche **dadurch gekennzeichnet, dass** die Reinigungslösung hautberuhigende Hilfstoffe, bevorzugt Polydocanol, Panthenol, Nachtkerzenöl, Bisabolol, Kamillenextrakt enthält.

11. Reinigungsprodukt nach einem der vorangehenden Ansprüche **dadurch gekennzeichnet, dass** die Reinigungsformulierung 0,001- 5 Gew.% Kamilenextrakt enthält.

12. Reinigungsprodukt nach einem der vorangehenden Ansprüche **dadurch gekennzeichnet, dass** die Reinigungszubereitung weitere kosmetische Hilfsstoffe enthält, insbesondere Emolients, Ölkomponenten, Farbstoffe, Pigmente, Konservierungsmittel und Parfüm.

13. Reinigungsprodukt nach einem der vorangehenden Ansprüche **dadurch gekennzeichnet, dass** die Reinigungsformulierung hydrierte Fettsäuremonoglyceride, Fettsäurediglyceride und/oder Fettsäuretriglyceride enthält, die ethoxyliert wurden und einen Ethoxylierungsgrad von 20 bis 500 aufweisen.

14. Reinigungsprodukt nach einem der vorangehenden Ansprüche **dadurch gekennzeichnet, dass** die Reinigungsformulierung eine Viskosität von 1 bis 1500 mPas aufweist gemessen mit Viskotester der Firma Thermo Haake GmbH, Modell Viskotester VT 02, Drehkörper Typ 1, Messung analog zu DIN 53019 und DIN 168.

15. Reinigungsprodukt nach einem der vorangehenden Ansprüche **dadurch gekennzeichnet, dass** die Reinigungsformulierung einphasig ist.

16. Verwendung einer Reinigungszubereitung nach einem oder mehreren der vorhergehenden Ansprüche zur Reinigung der Vaginalschleimhaut.

## Claims

1. Cosmetic cleaning product comprising
a) a liquid cleaning formulation with a pH of less than 5.0, comprising:
- at least one anionic surfactant,
- one or more nonionic surfactants,
- 0.001-5% lactic acid,
b) a packaging system in the form of a pump foamer comprising a storage container and a pump mechanism designed as cloture and with riser tube for the foaming of the surfactant solution located in the storage container, **characterized in that** the anionic surfactant is selected from the group of acyl glutamates.

2. Cleaning product according to Claim 1, **characterized in that** the pump foamer has a splashproof design.

3. Cleaning product according to one of the preceding claims, **characterized in that** the pump foamer produces foam on an incorporated mesh upon removal.

4. Cleaning product according to one of the preceding claims, **characterized in that** the nonionic surfactant is selected from the group of the alkyl glucosides.

5. Cleaning product according to one of the preceding claims, **characterized in that** lauryl glucoside and/or decyl glucoside is selected as nonionic surfactant.

6. Cleaning product according to one of the preceding claims, **characterized in that** sodium cocoyl glutamate is selected as anionic surfactant.

7. Cleaning product according to one of the preceding claims, **characterized in that** the surfactant concentration is in the range from 2 to 15% by weight, based on the total weight of the formulation, in particular in the range from 4 to 9% by weight.

8. Cleaning product according to one of the preceding claims, **characterized in that** the ratio of the anionic surfactant to the nonionic surfactant as a:b - where a and b are rational numbers between 2 and 5 - is preferably the ratios 5:3 to 3.5:4 and very particularly 2.0:3.

9. Cleaning product according to one of the preceding claims, **characterized in that** the cleaning solution comprises sodium cocoyl glutamate and decyl glucoside in a ratio of from 4:1 to 0.5:1, preferably from 3:1 to 0.5:1, particularly preferably from 2:1 to 0.7:1.

10. Cleaning product according to one of the preceding claims, **characterized in that** the cleaning solution contains skin-calming auxiliaries, preferably polydocanol, panthenol, evening primrose oil, bisabolol, camomile extract.

11. Cleaning product according to one of the preceding claims, **characterized in that** the cleaning formulation comprises 0.001-5% by weight of camomile extract.

12. Cleaning product according to one of the preceding claims, **characterized in that** the cleaning preparation comprises further cosmetic auxiliaries, in particular emollients, oil components, dyes, pigments, preservatives and perfume.

13. Cleaning product according to one of the preceding claims, **characterized in that** the cleaning formulation comprises hydrogenated fatty acid monoglycerides, fatty acid diglycerides and/or fatty acid triglycerides which have been ethoxylated and have a degree of ethoxylation of from 20 to 500.

14. Cleaning product according to one of the preceding claims, **characterized in that** the cleaning formulation has a viscosity of from 1 to 1500 mPas, measured using Viscotester from Thermo Haake GmbH, model Viscotester VT 02, rotating body type 1, measurement analogous to DIN 53019 and DIN 168.

15. Cleaning product according to one of the preceding claims, **characterized in that** the cleaning formulation is single-ptiase.

16. Use of a cleaning preparation according to one or more of the preceding claims for cleaning the vaginal mucosa.

## Revendications

1. Produit cosmétique de nettoyage, comprenant
a) une formulation de nettoyage liquide présentant un pH inférieur à 5,0, contenant :
- au moins un agent tensioactif anionique,
- un ou plusieurs agents tensioactifs non ioniques,
- 0,001-5% d'acide lactique,
b) un système d'emballage sous forme d'un dispositif de moussage à pompe contenant un réservoir et un mécanisme de pompe réalisé sous forme d'une obturation avec un tube ascendant pour le moussage de la solution tensioactive se trouvant dans le réservoir,
**caractérisé en ce que** l'agent tensioactif anionique est choisi dans le groupe des glutamates d'acyle.

2. Produit de nettoyage selon la revendication 1, **caractérisé en ce que** le dispositif de moussage à pompe est réalisé de manière à être protégé contre les projections d'eau.

3. Produit de nettoyage selon l'une quelconque des revendications précédentes, **caractérisé en ce que** le dispositif de moussage à pompe produit de la mousse lors du prélèvement au niveau d'une toile incorporée.

4. Produit de nettoyage selon l'une quelconque des revendications précédentes, **caractérisé en ce que** l'agent tensioactif non ionique est choisi dans le groupe des alkylglucosides.

5. Produit de nettoyage selon l'une quelconque des revendications précédentes, **caractérisé en ce qu'**on choisis comme agent tensioactif non ionique du laurylglucoside et/ou du décylglucoside.

6. Produit de nettoyage selon l'une quelconque des revendications précédentes, **caractérisé en ce qu'**on choisit comme agent tensioactif anionique du cocoylglutamate de sodium.

7. Produit de nettoyage selon l'une quelconque des revendications précédentes, **caractérisé en ce que** la concentration en agent tensioactif est située dans la plage de 2 à 15% en poids par rapport au poids total de la formulation, en particulier dans la plage de 4 à 9% en poids.

8. Produit de nettoyage selon l'une quelconque des revendications précédentes, **caractérisé en ce que** le rapport d'agent tensioactif anionique à non ionique correspond à a:b - avec a et b représentant un nombre rationnel entre 2 et 5 - de préférence le rapport 5:3 à 3,5:4 et de manière tout particulièrement préférée de 2,0:3.

9. Produit de nettoyage selon l'une quelconque des revendications précédentes, **caractérisé en ce que** la solution de nettoyage contient du cocaylglutamate de sodium et du décylglucoside dans un rapport de 4:1 à à 0,5:1, de préférence de 3:1 à 0,5:1, de manière particulièrement préférée de 2:1 à 0,7:1.

10. Produit de nettoyage selon l'une quelconque des revendications précédentes, **caractérisé en ce que** la solution de nettoyage contient des adjuvants calmant la peau, de préférence le polydocanol, le panthénol, l'huile d'onagre, le bisabolol, l'extrait de camomille.

11. Produit de nettoyage selon l'une quelconque des revendications précédentes, **caractérisé en ce que** la formulation de nettoyage contient 0,001-5% en poids d'extrait de camomille.

12. Produit de nettoyage selon l'une quelconque des revendications précédentes, **caractérisé en ce que** la préparation de nettoyage contient d'autres adjuvants cosmétiques, en particulier des émollients, des composants huileux, des colorants, des pigments, des conservateurs et du parfum.

13. Produit de nettoyage selon l'une quelconque des revendications précédentes, **caractérisé en ce que** la composition de nettoyage contient des monoglycérides d'acides gras hydrogénés, des diglycérides d'acides gras et/ou des triglycérides d'acides gras, qui ont été éthoxylés et qui présentent un degré d'éthoxylation de 20 à 500.

14. Produit de nettoyage selon l'une quelconque des revendications précédentes, **caractérisé en ce que** la formulation de nettoyage présente une viscosité de 1 à 1500 mPa.s, mesurée avec le Viskotester de la société Thermo Haake GmbH, modéle Viskotester VT 02, corps rotatif de type 1, mesure selon la norme DIN 53019 et DIN 168.

15. Produit de nettoyage selon l'une quelconque des revendications précédentes, **caractérisé en ce que** la formulation de nettoyage est à une phase.

16. Utilisation d'une composition de nettoyage selon l'une ou plusieurs des revendications précédentes pour le nettoyage de la muqueuse vaginale.
